# EUROPEAN PATENT SPECIFICATION

(11) **EP 2 488 488 B1**
(45) Date of publication and mention of the grant of the patent: **20.11.2013**
(21) Application number: 10768127.2
(22) Date of filing: 07.10.2010
(51) Int. Cl.: C11D 1/00, C11D 3/00, C11D 3/48, C11D 11/00, A62D 1/00, A62D 1/02

(54) **METHODS USING AMPHOTERIC SURFACTANTS**
VERFAHREN ZUR VERWENDUNG AMPHOTERER TENSIDE
PROCÉDÉS METTANT EN OEUVRE DES TENSIOACTIFS AMPHOTÈRES

(30) Priority: 15.10.2009 US 579488
(43) Date of publication of application: 22.08.2012
(73) Proprietor: E. I. du Pont de Nemours and Company, Wilmington, DE 19898 (US)
(72) Inventor: PENG, Sheng, Hockessin Delaware 19707 (US); YAKE, Allison, Mary, Landenberg Pennsylvania 19350 (US)
(74) Representative: Dannenberger, Oliver Andre
(86) International application number: PCT/US2010/051742
(87) International publication number: WO 2011/046795

(56) References cited:
- WO-A2-2008/027604
- US-A- 3 613 787
- US-A- 3 721 706
- US-A1- 2009 137 773
- US-B1- 7 385 077

## Description

### FIELD OF THE INVENTION

This invention relates to use of amphoteric fluorinated sulfonate compounds which contain at least one vinylidene fluoride or oxygen moiety in methods to provide surface effects in several applications, and is particularly suitable for oilfield applications and for coating applications.

### BACKGROUND OF THE INVENTION

Fluorinated sulfonates are useful as surfactants in various applications. Commercially available fluorinated surfactants usually contain a perfluoroalkyl terminal chain. Honda, et al., in "Molecular Aggregation Structure and Surface Properties of Poly(fluoroalkylacrylate) Thin Films" Macromolecules (2005), 38(13), 5699-5705, disclose that a perfluoroalkyl chain of at least 8 carbons is necessary to maintain the perfluoroalkyl chains in a parallel orientation. For such perfluoroalkyl chains containing less than 8 continuous perfluorinated carbons, a reorientation occurs which decreases or eliminates the ability for exhibiting desirable surface properties. Thus longer perfluoroalkyl chains which contain a higher fluorine content at a given concentration typically provide better performance. However, the fluorinated materials derived from longer perfluoroalkyl chains are more expensive. Reducing the fluorine content with delivery of the same or better performance is therefore desirable.

U.S. Patent 6,201,122, discloses a fluoroaliphatic radical-containing sulfonamido anionic compound, wherein the fluoroaliphatic radical group contains 3 to 20 carbons, and is preferably CₙF₂ₙ₊₁ therein n is 4 to 10. The compounds are useful as anionic surfactants in liquid systems. However, anionic surfactants are known to precipitate out of formulations in certain end use applications, such as formulations commonly used in fire fighting applications and oilfield applications.

U.S. Patent application US 2009/137773 A1, discloses compounds having fluorinated branched alkyl ammonium sulphonamide moieties being useful as surfactants, for example in fire-fighting foams as emulsifiers and wetting agents.

It is desirable to have methods of imparting surface effects using surfactants containing partially fluorinated or shorter fluorinated terminal groups to achieve equivalent or improved surface performance at lower expense. It is also desirable to have such methods useful in a variety of applications without precipitation from the formulation employed. The present invention provides such methods.

### SUMMARY OF THE INVENTION

The present invention comprises a method of providing a surface effect to a medium or substrate comprising contacting the medium or substrate with a compound of formula (I): 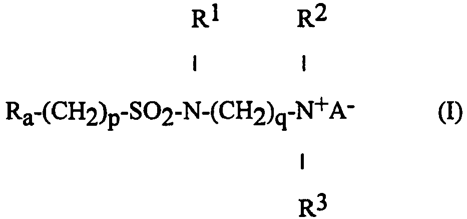 wherein
Rₐ is linear F(CF₂)ₙ(CH₂CF₂)ₘ-, or linear F(CF₂)ᵣ-O-B-;
B is (CₛF₂ₛ) optionally interrupted by 1 to 2 catenary oxygen atoms, each oxygen bonded to two carbon atoms,
n is 2 to 4, m is 1 to 4, r is 1 to 4, s is 1 to 4, provided that (r + s) is a maximum of 7,
A is O or (CH₂)ₖ-COO,
R¹ is hydrogen or a methyl,
R² and R³ are each independently alkyl having 1 to 6 carbon atoms, and
p, q and k are each independently integers from 1 to 10.

The present invention further comprises a method of lowering surface tension of an aqueous medium comprising contacting the medium with a compound of formula (I) as defined above.

### DETAILED DESCRIPTION

Trademarks are shown herein in upper case.

The present invention comprises a method of providing a surface effect to a medium or substrate comprising contacting the medium or substrate with a compound of formula (I) as defined above.

The present invention comprises a method of reducing surface tension of an aqueous medium at low concentrations employing compounds that have amphiphilic properties. By "amphiphilic" is meant that the compounds are attracted to different kinds of media.

The methods of the present invention use a compound of formula (I): 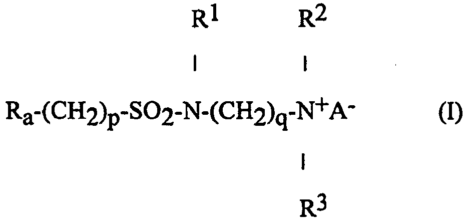 wherein
Rₐ is linear F(CF₂)ₙ(CH₂CF₂)ₘ-, or linear F(CF₂)ᵣ-O-B-;
B is (CₛF₂ₛ) optionally interrupted by 1 to 2 catenary oxygen atoms, each oxygen bonded to two carbon atoms,
n is 2 to 4, m is 1 to 4, r is 1 to 4, s is 1 to 4, provided that (r + s) is a maximum of 7,
A is O or (CH₂)ₖ-COO,
R¹ is hydrogen or a methyl,
R² and R³ are each independently alkyl having 1 to 6 carbon atoms, and
p, q and k are each independently integers from 1 to 10.

Preferred compounds of formula (I) for use in the methods of the present invention are those wherein Rₐ is F(CF₂)ₙ(CH₂CF₂)ₘ- wherein n is 1 to 4, and m is 1 to 3, preferably 2. Also preferred are those compounds of formula (I) wherein Rₐ is F(CF₂)ᵣ-O-B wherein r is 2 to 4, preferably 3 to 4, and B is (CₛF₂ₛ) wherein s is 2 to 3, preferably 2.

The compounds of formula (I) are prepared from an intermediate amine of the formula (II): 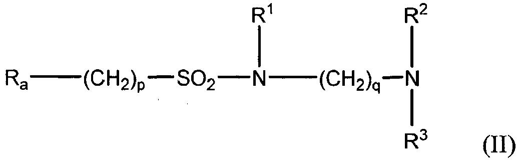 wherein Rₐ, p, q, R¹, R², and R³ are the same as defined above in formula (I). Compounds of formula (II) are reacted with alpha-ethylenic acids, aliphatic lactones or beta-halo-carboxylic acids to produce the compounds of formula (I). For example, the intermediate amine in formula (II) is reacted with sodium chloroacetate at temperature of about 78 °C. for about 24 hours to produce compounds of formula (I) wherein A is (CH₂)ₖ-C(O)O. Alternatively, the intermediate amine in formula (II) is oxidized to produce the compounds of Formula (I) wherein A is O. For example, the intermediate amine of formula (II) is reacted with hydrogen peroxide at a temperature of about 50 °C. for about 56 hours, followed by a second addition of hydrogen peroxide, and the reaction is maintained at about 50 °C for an extra 12 hours to produce compounds of formula (I) wherein A is O.

The intermediate amine of formula (II) can be synthesized by reacting an amine, preferably diaminopropylamine, with a fluorinated sulfonyl chloride of formula (III)

Rₐ-(CH₂)ₚ-SO₂Cl (III)

wherein Ra and p are as defined in formula (I).

The fluorinated sulfonyl chloride of formula (III) is formed by reacting a fluorinated thiocyanate of formula (IV)

Rₐ-(CH₂)ₚ- SCN (IV)a

wherein Ra and p are as defined in formula (I), with chlorine and acetic acid at about 45°C to 50°C.

The fluorinated thiocyanate of formula (IV) is prepared by reacting ethylene iodides of formula (V)

Rₐ-(CH₂)ₚ-I (V)

with potassium thiocyanate and trioctylmethylammonium chloride at 90 °C.

The ethylene iodides of formula (V) are prepared by reacting fluorinated iodides of formula (VI).

Rₐ -I (VI)

wherein Rₐ is as defined above in formula (I), with ethylene by the procedures described in U.S. Patent 3,979,469, (Ciba-Geigy, 1976).

Fluorinated iodides of formula (VI) having the formula F(CF₂)ₙ(CH₂CF₂)ₘI are produced by the known telomerization of vinylidene fluoride (VDF) with linear or branched perfluoroalkyl iodides. For example, see Balague, et al, "Synthesis of fluorinated telomers, Part 1, Telomerization of vinylidene fluoride with perfluoroalkyl iodides", J. Fluor. Chem. (1995), 70(2), 215-23. Preferred examples of iodides needed to make compounds of formula (I) wherein Rₐ is F(CF₂)ₙ(CH₂CF₂)ₘ- include F(CF₂)₄(CH₂CF₂)I and F(CF₂)₄(CH₂CF₂)₂I.

Fluorinated iodides of formula (VI) having the formula F(CF₂)ᵣ-O-B-wherein B is as defined above can be prepared from perfluoroalkyl ether iodides which can be made by the procedure described in US Patent 5,481,028. Preferred is the process in Example 8. This patent discloses the preparation of perfluoroalkyl ether iodides from perfluoro-n-propyl vinyl ether. Preferred examples of iodides needed to make compounds of formula (I) wherein Rₐ is F(CF₂)ᵣ-O-B- are F(CF₂)₃O(CF₂)₂I, F(CF₂)₂O(CF₂)₄I, and F(CF₂)₄O(CF₂)₂I.

In a preferred embodiment of this invention, the methods employ a surfactant of formula (I) having the following specific formula: 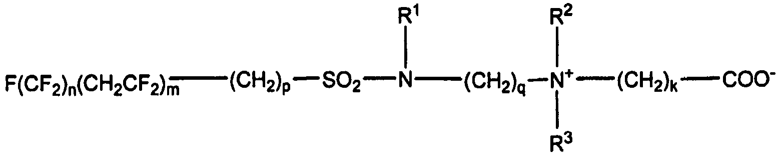 wherein m is 1 to 4 and n, p, k, R¹, R², and R³ are as defined above in formula (I).

In a further preferred embodiment of this invention, the methods employ a surfactant of formula (I) having the following specific formula: 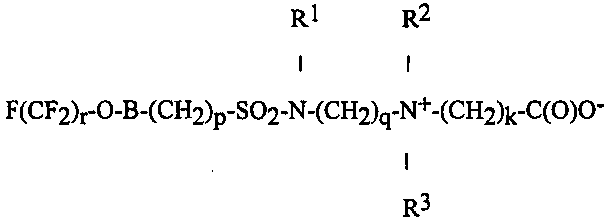 wherein r, B, p, q, k, R¹, R², and R³ are as defined above in formula (I).

In a further preferred embodiment of this invention, the methods employ a surfactant of formula (I) having the following specific formula: 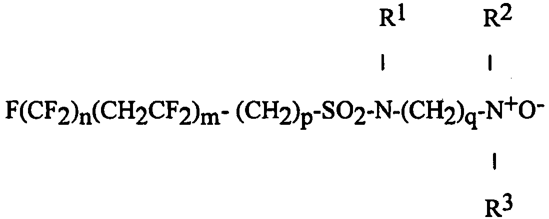 wherein m is 1 to 4 and n, p, k, R¹, R², and R³ are as defined above in formula (I).

In a further preferred embodiment of this invention, the methods employ a surfactant of formula (I) having the following specific formula: 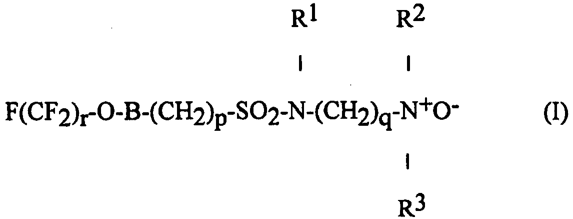 wherein r, B, p, q, R¹, R², and R³ are as defined above in formula (I)

The method of the present invention significantly reduce the surface tension of aqueous solutions at low concentrations. Uses include, but are not limited to, filming, foaming, wetting, leveling, spreading, stabilizing, dispersing and as emulsifying agents.

One embodiment of the present invention is a method of lowering surface tension comprising contacting an aqueous medium with a compound of formula (I) at low concentrations. This method is capable of lowering the surface tension of aqueous media to values less than 25 milli-newtons per meter, preferably less than 20 milli-newtons per meter, at a concentration of the surfactant in the medium of less than 0.5 % by weight, and preferably less than 0.2 % by weight, more preferably less than 0.1% by weight. These method is characterized by its efficiency in lowering the surface tension at low concentrations by selective adsorption on the interface, which is determined by the amphiphilic nature of the surfactants.

Any of a wide variety of media are suitable for use in the method of the present invention. Typically, the medium is a liquid. Examples of suitable medium include an aqueous solution, water, sea water, saline solution, hydrocarbon, halocarbon system, coating composition, latex, polymer, floor finish, ink, oil or gas field additive or stream, emulsifying agent, foaming agent, release agent, repellency agent, flow modifier, film evaporation inhibitor, wetting agent, penetrating agent, cleaner, grinding agent, electroplating agent, corrosion inhibitor, etchant solution, soldering agent, dispersion aid, microbial agent, pulping aid, rinsing aid, polishing agent, personal care composition, drying agent, antistatic agent, floor polish, or bonding agent. Adding a compound of formula (I) in the method of the present invention to the medium results in lowering the surface tension of the medium due to the surfactant properties of the compound. The compound of formula (I) is typically simply blended with or added to the medium. A concentration of about 0.1 % by weight of surfactant is sufficient to lower surface tension to less than 25 mN/m, preferably less than 20 nM/m.

Another embodiment of the present invention comprises a method of providing surface effects to a medium or substrate. The surface effect is provided by contacting a compound of formula (I) with the medium or substrate. Suitable media are as discussed above. For a coated substrate a compound of formula (I) is often added to a coating base prior to deposition on the substrate. Surface effects include, for example, in addition to lowering surface tension, leveling and wetting. "Leveling and wetting" as used herein refers to the uniformity of coverage of the coating when applied to a substrate. It is undesirable to have streaking, surface defects, or withdrawal of the coating from the substrate surface at the edges or otherwise. An even coating will provide a superior dried coating on the substrate surface.

Suitable coating compositions, referred to herein by the term "coating base", include a composition, typically a liquid formulation, of an alkyd coating, Type I urethane coating, unsaturated polyester coating, water-dispersed coating, a floor polish, or a floor finish, and is applied to a substrate for the purpose of creating a lasting film on the substrate surface. These are conventional paints, stains, and similar coating compositions.

By the term "alkyd coating" as used herein is meant a conventional liquid coating based on alkyd resins, typically a paint, clear coating, or stain. The alkyd resins are complex branched and cross-linked polyesters containing unsaturated aliphatic acid residues. Conventional alkyd coatings utilize, as the binder or film-forming component, a curing or drying alkyd resin. Alkyd resin coatings contain unsaturated aliphatic acid resides derived from drying oils. These resins spontaneously polymerize in the presence of oxygen or air to yield a solid protective film. The polymerization is termed "drying" or "curing" and occurs as a result of autoxidation of the unsaturated carbon-carbon bonds in the aliphatic acid component of the oil by atmospheric oxygen. When applied to a surface as a thin liquid layer of formulated alkyd coating, the cured films that form are relatively hard, non-melting, and substantially insoluble in many organic solvents that act as solvents or thinners for the unoxidized alkyd resin or drying oil. Such drying oils have been used as raw materials for oil-based coatings and are described in the literature.

By the term "urethane coating" as used hereinafter is meant a conventional liquid coating based on Type I urethane resins, typically a paint, clear coating, or stain. Urethane coatings typically contain the reaction product of a polyisocyanate, usually toluene diisocyanate, and a polyhydric alcohol ester of drying oil acids. Urethane coatings are classified by ASTM D-1 into five categories. Type I urethane coatings contain a pre-reacted autoxidizable binder as described in Surface Coatings Vol. I, previously cited. These are also known as uralkyds, urethane-modified alkyds, oil-modified urethanes, urethane oils, or urethane alkyds, are the largest volume category of polyurethane coatings and include paints, clear coatings, or stains. The cured coating is formed by air oxidation and polymerization of the unsaturated drying oil residue in the binder.

By the term "unsaturated polyester coating" as used hereinafter is meant a conventional liquid coating based on unsaturated polyester resins, dissolved in monomers and containing initiators and catalysts as needed, typically as a paint, clear coating, or gel coat formulation. Unsaturated polyester resins contain as the unsaturated prepolymer the product obtained from the condensation polymerization of a glycol such as 1,2- propylene glycol or 1,3-butylene glycol with an unsaturated acid such as maleic (or of maleic and a saturated acid, e.g., phthalic) in the anhydride form. The unsaturated prepolymer is a linear polymer containing unsaturation in the chain. This is dissolved in a suitable monomer, for instance styrene, to produce the final resin. The film is produced by copolymerization of the linear polymer and monomer by means of a free radical mechanism. The free radicals can be generated by heat, or more usually by addition of a peroxide, such as benzoyl peroxide, separately packaged and added before use. Such coating compositions are frequently termed "gel coat" finishes. For curing coatings at room temperature, the decomposition of peroxides into free radicals is catalyzed by certain metal ions, usually cobalt. The solutions of peroxide and cobalt compound are added separately to the mix and well stirred before application. The unsaturated polyester resins that cure by a free radical mechanism are also suited to irradiation curing using, for instance, ultraviolet light. This form of cure, in which no heat is produced, is particularly suited to films on wood or board. Other radiation sources, for instance electron-beam curing, are also used.

By the term "water-dispersed coatings" as used herein is meant coatings intended for the decoration or protection of a substrate composed of water as an essential dispersing component such as an emulsion, latex, or suspension of a film-forming material dispersed in an aqueous phase. "Water-dispersed coating" is a general classification that describes a number of formulations and includes members of the above described classifications as well as members of other classifications. Water-dispersed coatings in general contain other common coating ingredients. Water-dispersed coatings are exemplified by, but not limited to, pigmented coatings such as latex paints, unpigmented coatings such as wood sealers, stains, and finishes, coatings for masonry and cement, and water-based asphalt emulsions. A water dispersed coating optionally contains surfactants, protective colloids and thickeners, pigments and extender pigments, preservatives, fungicides, freeze-thaw stabilizers, antifoam agents, agents to control pH, coalescing aids, and other ingredients. For latex paints the film forming material is a latex polymer of acrylate acrylic, vinyl-acrylic, vinyl, or a mixture thereof. Such water-dispersed coating compositions are described by C. R. Martens in "Emulsion and Water-Soluble Paints and Coatings" (Reinhold Publishing Corporation, New York, NY, 1965).

By the term "dried coating" as used herein is meant the final decorative and/or protective film obtained after the coating composition has dried, set or cured. Such a final film can be achieved by, for non-limiting example, curing, coalescing, polymerizing, interpenetrating, radiation curing, UV curing or evaporation. Final films can also be applied in a dry and final state as in dry coating.

When used as additives to a coating base in the method of the present invention the compounds of Formla (I) as defined above are effectively introduced to the coating base or other composition by thoroughly stirring it in at room or ambient temperature. More elaborate mixing can be employed such as using a mechanical shaker or providing heat or other methods. Such methods are not necessary and do not substantially improve the final composition. When used as an additive to latex paints, the compositions of the invention generally are added at 0.001% by weight to 5% by weight by dry weight of the compound of formula (I) in the wet paint. Preferably from 0.01 % by weight to 1% by weight, and more preferably from 0.1% by weight to 0.5% by weight is used.

Floor waxes, polishes, or finishes (hereinafter "floor finishes") are generally water based or solvent based polymer emulsions. The method of the present invention is suitable for use for such floor finishes. Commercially available floor finish compositions typically are aqueous emulsion-based polymer compositions comprising one or more organic solvents, plasticizers, coating aides, anti-foaming agents, surfactants, polymer emulsions, metal complexing agents, and waxes. The particle size range and solids content of the polymer are usually controlled to control the product viscosity, film hardness and resistance to deterioration. Polymers containing polar groups function to enhance solubility and may also act as wetting or leveling agents providing good optical properties such a high gloss and distinctness of reflected image.

Preferred polymers for use in floor finishes include acrylic polymers, polymers derived from cyclic ethers, and polymers derived from vinyl substituted aromatics. Acrylic polymers include various poly(alkyl acrylates), poly(alkyl methacrylates), hydroxyl substituted poly(alkyl acrylates) and poly(alkyl methacrylates). Commercially available acrylic copolymers used in floor finishes include, for example, methyl methacrylate/butyl acrylate/methacrylic acid (MMA/BA/MAA) copolymers; methyl methacrylate/butyl acrylate/acrylic acid (MMA/BA/AA) copolymers, and the like. Commercially available styrene-acrylic copolymers include styrene/methyl methacrylate/butyl acrylate/methacrylic acid (S/MMA/BA/MMA) copolymers; styrene/methyl methacrylate/butyl acrylate/acrylic acid (S/MMA/BA/AA) copolymers; and the like. Polymers derived from cyclic ethers usually contain 2 to 5 carbon atoms in the ring with optional alkyl groups substituted thereon. Examples include various oxiranes, oxetanes, tetrahydrofurans, tetrahydropyrans, dioxanes, trioxanes, and caprolactone. Polymers derived from vinyl substituted aromatics include for example those made from styrenes, pyridines, conjugated dienes, and copolymers thereof. Polyesters, polyamides, polyurethanes and polysiloxanes are also used in floor finishes.

The waxes or mixtures of waxes that are used in floor finishes include waxes of a vegetable, animal, synthetic, and/or mineral origin. Representative waxes include, for example, carnuba, candelilla, lanolin, stearin, beeswax, oxidized polyethylene wax, polyethylene emulsions, polypropylene, copolymers of ethylene and acrylic esters, hydrogenerated coconut oil or soybean oil, and the mineral waxes such as paraffin or ceresin. The waxes typically range from 0 to 15 weight percent and preferably from 2 to 10 weight percent based on the weight of the finish composition.

When used as additives to a floor finish the compounds of formula (I) as defined above are effectively introduced to the composition by thoroughly stirring it in at room or ambient temperature. More elaborate mixing can be employed such as using a mechanical shaker or providing heat or other methods. When used as an additive to floor finishes, the compounds of formula (I) generally are added at 0.001% by weight to 5% by weight by dry weight of the compound of formula (I) in the wet composition. Preferably from 0.01 % by weight to 1% by weight, and more preferably from 0.1 % by weight to 0.5% by weight is used.

Floor waxes or polishes are water based, solvent based and polymer. The method of the present invention is suitable for use with any of these. Water-based and polymer waxes dry to a high gloss without buffing; solvent-based wax requires vigorous buffing. Water-based wax is recommended for asphalt, vinyl, vinyl asbestos and rubber-tiled floors; solvent-based waxes produce a hard, shiny finish and are best for wood, cork and terrazzo floors. Self-polishing waxes, such as polymer or resin, will yellow or discolor and wear off in heavy traffic areas; they should be stripped off and reapplied after three or four coats.

In another embodiment of the present invention the methods of the present invention are useful in gas and oil field applications. Herein a hydrocarbon is either a gas or oil product which is produced or recovered from a subterranean zone. A well or well bore is drilled and created to penetrate such a hydrocarbon containing subterranean zone. The method of the present invention is useful to provide a surfactant or foaming agent to modify and improve the wettability and surface conditions, such as the surface tension of the subterranean formation around the well bore, and is also useful to improve the permeability and flow rate to enhance oil well or gas well recovery and productivity.

The term "drill fluids" as used herein means those liquids that are added to a well or well bore penetrating a subterranean zone containing hydrocarbon or gas prior to or during a drilling operation. Examples can include water, brine, solvent, hydrocarbons, surfactants, oils, kerosene, fracturing fluids, stimulating fluids, oil-based drill muds, clay stabilizers, treatment fluids, and mixtures thereof.

The term "well fluids" as used herein means those liquids that occur in or are added to a well or well bore penetrating a subterranean zone containing hydrocarbon or gas. Examples can include drill fluids, water, brine, solvent, hydrocarbons, surfactants, oils, kerosene, fracturing fluids, stimulating fluids, oil-based drill muds, clay stabilizers, treatment fluids, and mixtures thereof.

The term "liquid treatment stream or gas treatment stream" as used herein means a liquid composition or gas composition, or a combination thereof, injected into a well penetrating a subterranean zone containing hydrocarbon or gas, or into a well bore area, in the operation of extracting the hydrocarbon or gas. Examples can include steam, drill fluids, well fluids, stimulating fluids, water, brine, solvent, hydrocarbons, surfactants, fracturing fluids, oil-based drill muds, clay stabilizers, treatment fluids, and mixtures thereof.

The method of the present invention provides a compound of formula (I), which acts as a surfactant or foaming agent for oil field and gas field applications. In this embodiment the compound of formula (I) is typically used in an aqueous medium or solvent medium selected from the group consisting of water, saline solution, KCl solution, HCl solution, hydrocarbon, halocarbon, drill fluids, well fluids, liquid treatment stream, gas treatment stream, and a mixture thereof. The method of the present invention is useful to provide an additive in drill fluids, well fluids, and other treatment fluids for subterranean formations, to enhance gas or oil recovery by altering surface tension, wettability, or viscosity of the fluids, oils, condensates, and muds employed or encountered in such operations. The surfactant can be used for foaming porous rock or soil medium of a subterranean formation, or for other known well or well bore treatments.

The present invention provides a surfactant or foaming fluid which comprises the compounds of formula (I) and a medium, wherein the compound of formula (I) is present at a concentration range of from 0.001 % to 50% by weight, preferably a range of from 0.01% to 30% by weight, and more preferably a range of from 0.05% to 20% by weight in the medium.

The present invention comprises a method of lowering the surface tension within a subterranean formation containing hydrocarbons comprising adding a compound of formula (I) as described above to a medium which is a carrier contacted with the subterranean formation. One method of contacting is injection of the carrier or medium into the subterranean formation, for example by using a downhole, well, or well bore. The compound of formula (I) is added to a carrier or medium such as a fluid or gas which will be in contact with the subterranean formation during operations to remove oil or gas from the formation. Examples include drill fluids, well fluids, stimulation fluids, liquid treatment stream, gas treatment stream, fractionating fluids, clay stabilizers, or other liquids or gases employed when extracting the hydrocarbons from the formation. The methods of the present invention employing compounds of formula (I) can be used in one or more of a pretreatment stage of injection of a pre-flush of various liquids, or in matrix or stimulation activities; in the main stage in various carrier fluids, or in a soaking of the formation for a specific time period; or in a post treatment stage for displacement operation to achieve better placement of the fluids containing the surfactant composition. The compound of formula (I) is used in the media in the form of a liquid, emulsion, dispersion, or foaming agent.

Foaming is a desirable property of the surfactants used in the method of the present invention when used as additives to drill fluids, well fluids, and other fluids in oil and/or gas field applications for enhanced production and recovery. The aqueous or solvent based drilling fluids, well fluids, liquid or gas treatment streams, or other carrier compositions which contain the compound of formula (I) foam during drilling or well treatment processes, and therefore provide advantages for enhanced production and recovery. Examples of such advantages from the surfactant and foaming properties include aiding in the removal of fines from the well around the drill-bit and wellbore treatment area, and adjusting the permeability and wettability properties where the fluids contact around the drill-bit and wellbore treatment area. The addition of the surfactant using the method of the present invention boosts the foaming properties of the oil/gas well drilling fluids and treatment fluids. If these fines are not efficiently removed, they can result in damage to the drill-bit head, costing time and money to replace or repair. In addition the method of the present invention is useful to reduce the viscosity of the hydrocarbon to permit easier extraction.

Another advantage of contacting a subterranean formation containing hydrocarbons with the surfactant of formula (I) as defined above, is providing a method for stimulating production of hydrocarbons during operations to extract hydrocarbons from a subterranean formation. The method of the present invention employs the surfactant compounds of formula (I) as stimulation fluid additives for stimulation activities, such as hydraulic fracturing and acidizing. In these situations the stable foams of the surfactants improve the wetting of the stimulation fluid on the formation surface (rock) to allow for deeper penetration and better stimulation of the well bore region. The low surface tension of these additives permit the stimulation fluids to be more efficiently and easily recovered from downhole using the method of the present invention. As a result, the well will be able to more effectively produce gas and oil.

The method of the present invention is further useful to provide an aid to prevent and remedy water blocks or condensate blocks in wells and well bore areas. It is known that water can accumulate near the well bore of an oil or gas well and decrease productivity by decreasing the relative permeability of the oil or gas, which is called water block. In addition liquid hydrocarbons can also accumulate and cause a decrease in productivity in gas wells near or far from the well bore region known as condensate block. The compounds used in the method of the present invention can be used to help in removal of at least a portion of such accumulations of liquids in a water block or condensate block, or for reducing or preventing the formation of the accumulation of liquids in such blocks. The surfactant employed in the method of the present invention is useful as a surfactant additive in drill fluids, well fluids and treatment fluids for subterranean formation to alter the wettability and permeability by its surface active properties. Such surfactants, for example, are used within the porous rock medium of subterranean formation and can result in pressure changes or as foams can block the gas drain paths and result in the oil/gas recovery increases.

The methods of the present invention have several uses and advantages as detailed above. The methods provide surface effects to media and substrates, such a lowering of surface tension, leveling and wetting, and foaming. The compounds of formula (I) employed in the methods of the present invention do not precipitate out of formulations commonly used in oilfield applications or other applications. The compounds employed in the methods of the present invention have a shorter terminal perfluoroalkyl group present, which is more economical than longer chain perfluoroalkyls due to the reduction in fluorine present, but still provide comparable or superior performance.

### Test Methods and Materials

The following test methods and materials (intermediates) were used in the Examples herein. Proton and ¹⁹F NMR as well as electrospray mass spectroscopy was used to confirm compositions of the intermediates and Examples.

### Test Methods

### Test Method 1-Surface Tension Measurement

The surface tension of the examples was measured via a Kruess Tensiometer, K11 Version 2.501, in accordance with instructions with the equipment. The Wilhelmy Plate method was used. A vertical plate of known perimeter was attached to a balance, and the force due to wetting was measured. Ten replicates were tested of each dilution, and the following machine settings were used: Plate Method SFT, 1.0 sec interval, 40.2 mm wetted length, 10 reading limit, 2 dynbes/cm min Standard Deviation, and 9.80665 m/s² Gr. Acc. Lower surface tension indicated superior performance.

A stock solution was prepared for the highest concentration of fluorosurfactant example to be analyzed. The concentration of the solutions was by percent active ingredient, weight percent or fluorine content. This stock solution was prepared in deionized water, in a floor polish (RHOPLEX® 3829, Formulation N-29-1 available from Rohm & Haas, Philadelphia, PA), in 2% KCl in water, or in 15% HCl in water depending on the desired application for which the surface tension was being measured. The stock solution was stirred overnight (for approximately 12 hours) to ensure complete mixing. Additional concentrations of the fluorosurfactant example for analysis were made by diluting the stock solution according to the equation MᵢVᵢ = M_{f}V_{f}, where Mᵢ is the concentration of the stock solution, M_{f} is the concentration of the final solution, V_{f} is the final volume of the sample, and Vᵢ is the volume of the stock solution that is needed in order to formulate the final sample. The concentration dilution samples were shaken thoroughly and then left to sit undisturbed for 30 minutes. These samples were then mixed and poured into a small container. Solutions of 2% KCl and 15% HCl were typically used in the surface tension measurements for oilfield applications because they mimic the stimulation fluid types that are pumped down hole into wells. The 2% KCl solution was similar to the salinity of the fracture fluids that are used to hydraulically fracture a well. The 15% HCl solution emulated the acidizing stimulation treatment fluid that is used to help dissolve the formation rock in wells. The floor polish was used for applications in the consumer, institutional, and industrial segments for demonstration of providing surface effects to substrates. The surface tension was measured using a Kruess Tensiometer, K11 Version 2.501 in accordance with instructions with the equipment as described above. Lower surface tension indicated superior performance.

### Test Method 2- Leveling and Wetting

To test the performance of the samples in their wetting and leveling ability, the following examples were added to a floor polish (RHOPLEX 3829, Formulation N-29-1, available from Rohm & Haas, Philadelphia, PA) and applied to half of a thoroughly cleaned 12 inch X 12 inch (30.36 cm X 30.36 cm) vinyl tile (available from Interfuse Vinyl Tiles by Estrie, Sherbrooke, QC Canada). The tiles were thoroughly cleaned by wetting the tiles, adding a powdered oxygen bleach cleanser and scrubbing using a green SCOTCH-BRITE scouring pad, available from 3M Company, St. Paul MN). This scrubbing procedure was used to remove the pre-existing coating on the tiles. The tiles initially had a uniform shiny finish; a uniform dull finish indicated coating removal. The tiles were then air-dried overnight. A 1 % by weight solution of the surfactant to be tested was prepared by dilution in deionized water. Following the resin manufacturer protocols, a 100 g portion of the RHOPLEX 3829 formulation was prepared, followed by addition of 0.75 g of the 1 % by weight surfactant solution, to provide a test floor polish.

The test floor polish was applied to the tile by placing 3 mL portion of the test polish in the center of the tile, and spreading from top to bottom using a cheesecloth applicator, and finally placing a large "X" across the tile, using the applicator. The "X" subsequently provided visual evidence of leveling at the rating step. The applicator was prepared from a two-layer 18 x 36 inch (46 x 91 cm) sheet of cheesecloth (from VWR, West Chester PA), folded twice into an eight-layer pad. One corner of the pad was then used as the applicator. The tile was allowed to dry for 30 min. and a total of 5 coats (Coating #s 1 - 5) were applied and dried, with the X test performed after each coating had been dried.

**Table 1**

| Visual Tile Rating Scale for Leveling | |
|---|---|
| Score | Description |
| 1 | Uneven surface coverage of the film, significant streaking and surface defects |
| 2 | Numerous surface defects and streaks are evident but, generally, film coats entire tile surface |
| 3 | Visible streaking and surface defects, withdrawal of the film from the edges of the tile |
| 4 | Minor surface imperfections or streaking |
| 5 | No visible surface defects or streaks |

### Test Method 3-Foaming

The test procedure used to evaluate the foaming was a modified version of the blender foaming test ASTM D3519-88. A blender, graduated cylinder, glass sample bottles and a stop watch were employed. First, stock solutions of the testing base solutions were made. These solutions were hard water, tap water, deionized water, or artificial sea water. Samples of 100 mL of the fluorosurfactant at 0.1 % active ingredient in the desired base testing solution were prepared and stirred overnight to ensure complete mixing. The blender was cleaned with deionized water, then acetone, and then de-ionized water again. Once clean, the blender was assembled for use. The test fluid sample of 100 mL was poured into the blender jar. The temperature of the test fluid was measured with a thermometer and recorded. The blender was then run for 20 seconds at 50-60% power. After 20 seconds, the liquid and foam were immediately poured into a 500 mL graduated cylinder. The initial liquid and foam height were measured in mL. The liquid and foam height were again measured at 5, 10 and 15 minutes. During this time, any observations of the foam were recorded such as its density or persistency. The blender foaming test was used to measure the amount of foam produced and the persistency of the foam. A difference in foam height of up to 10 mL is produced by variation in this method.

### Materials

### Intermediate 1

C₃F₇OCF₂CF₂I (100 g, 0.24 mol) and benzoyl peroxide (3 g) were charged to a pressure vessel under nitrogen. A series of three vacuum/nitrogen gas sequences was then executed at -50 °C and ethylene (18 g, 0.64 mol) was introduced. The vessel was heated for 24 hour at 110 °C. The autoclave was cooled to 0 °C and opened after degassing. Then the product was collected in a bottle. The product was distilled resulting in 80 g of C₃F₇OCF₂CF₂CH₂CH₂I in 80% yield. The boiling point was 56∼60 °C. at 25 mm Hg (3333 Pa).

Potassium thiocynate (21.34 g, 0.22 mol) was added to a mixture of C₃F₇OCF₂CF₂CH₂CH₂I (50 g, 0.11 mol) and trioctylmethylammonium chloride (0.2222 g) in 50 g of water. The reaction was heated overnight at 90 °C. After phase separation, the product C₃F₇OCF₂CF₂CH₂CH₂SCN was distilled as a colorless liquid (32 g, 78%).

Chlorine gas (132 g, 1.86 mol) and water (47 g, 2.6 mol) were fed into a mixture of C₃F₇OCF₂CF₂CH₂CH₂SCN (231 g, 0.62 mol) and acetic acid (130 g, 2.17 mol) over 10 hours at 45-50°C in an autoclave. A further 10 g of chlorine was added over 3 hours at 45 °C and heated at this temperature for 1 hour. The product was heated in a flask with a stir bar at 70 °C and 149 mL of hot water (70°C) was added. The organic layer was separated, followed by adding of toluene (125 g). The product in toluene was washed with 3.5% solution of brine (149 mL) at 70 °C twice. After the second wash, a Dean-Stark strap was set up to strip off water. The final product was 70% of C₃F₇OCF₂CF₂CH₂CH₂SO₂Cl (228 g, 90%) by weight in toluene.

C₃F₇OCF₂CF₂CH₂CH₂SO₂Cl (100 g, 0.242 mol, 66.8% in toluene) was added dropwise to a mixture of dimethylaminopropylamine (DMAPA) at 45 °C. After the addition, the reaction was heated at 75 °C overnight. The reaction mass was filtered and the wet cake was washed with 60 °C toluene. After stripping off the toluene, the concentrated organic product was washed with 200 mL of 95 °C deionized water. The product Intermediate 1, C₃F₇OCF₂CF₂CH₂CH₂SO₂N(H)-CH₂CH₂CH₂N(CH₃)₂ (101 g, 87.2%) was obtained as an amber colored solids after removing water under reduced pressure.

### Intermediate 2

Ethylene (25 g, 0.53 mol) was introduced to an autoclave charged with C₄F₉CH₂CF₂I (217 g, 0.87 mol) and d-(+)-limonene (1 g), and then the reactor was heated at 240 °C for 12 hours. The product, C₄F₉CH₂CF₂CH₂CH₂I, was obtained via vacuum distillation 81∼91 °C at 19∼24 mmHg in 62 % yield. Potassium thiocynate (21.34 g, 0.22 mol) was added to a mixture of C₄F₉CH₂CF₂CH₂CH₂I (50 g, 0.11 mol) and trioctylmethylammonium chloride (0.2222 g) in 50 g of water. The reaction was heated overnight at 90 °C. After phase separation, the product C₄F₉CH₂CF₂CH₂CH₂SCN was distilled as a colorless liquid (38 g, 95%).

Chlorine gas (118 g, 1.66 mol) and water (40 g, 2.22 mol) were fed into a mixture of C₄F₉CH₂CF₂CH₂CH₂SCN (205 g, 0.56 mol) and acetic acid (109 g, 1.82 mol) over 10 hours at 45∼50°C in an autoclave. The product was heated in a flask with a stir bar at 70 °C and hot water (70°C) was added. The organic layer was separated, followed by adding of toluene (216.25 g). The product in toluene was washed with 3.5% solution of brine at 70 °C twice. After the second wash, a Dean-Stark strap was set up to strip off water. The final product was 70% of C₄F₉CH₂CF₂CH₂CH₂SO₂Cl (228 g, 39%) by weight in toluene.

C₄F₉CH₂CF₂CH₂CH₂SO₂Cl (100 g, 0.23 mol, 70.3% in toluene) was added dropwise to a mixture of dimethylaminopropylamine (DMAPA)at 45 °C. After the addition, the reaction was heated at 75 °C overnight. The reaction mass was filtered and the wet cake was washed with 60 °C toluene. After stripping off the toluene, the concentrated organic product was washed with 200 mL of 95 °C deionized water. The product Intermediate 2, C₄F₉CH₂CF₂CH₂CH₂SO₂N(H)-CH₂CH₂CH₂N(CH₃)₂ (106 g, 96.8%) was obtained as a brown colored solids after removing water under reduced pressure.

### Intermediate 3

C₄F₉(CH₂CF₂)₂I (327 g, 0.69 mol) was charged to a Hastalloy C shaker tube reactor followed by a series of three vacuum/N₂ gas sequences. Ethylene (35 g, 2.57 mol) was introduced and the vessel heated to 240 °C for 3 hours, maintaining a pressure of 250 psig. Vacuum distillation of 2 combined runs provided 572 g (83%) of product, C₄F₉(CH₂CF₂)₂CH₂CH₂I, with boiling point 111∼120 °C at 16∼20 mmHg.

The flask was charged with C₄F₉(CH₂CF₂)₂CH₂CH₂I (500 g, 0.996 mol), potassium thiocyanate (194 g, 1.99 mol) and trioctylmethylammoniumchloride (ALIQUAT 336) (4.02 g, 0.00995 mol) under nitrogen. Deionised water (500 g, 27.8 mol) was added and the reaction mixture was heated to 90 °C for 18 hours. The organic layer was separated in a glass separating funnel and washed with hot (70 °C) deionised water. The product was distilled on a high vacuum system resulting in 407 g (94.3%) of C₄F₉(CH₂CF₂)₂CH₂CH₂SCN.; bp 129∼133 °C/ 1.0 mmHg.

An autoclave was charged with C₄F₉(CH₂CF₂)₂CH₂CH₂SCN (269 g, 0.62 mol) and acetic acid (130 g, 2.17 mol) under nitrogen and heated to 45∼50 °C. Chlorine gas (132 g, 1.86 mol) was fed at an estimated rate for 10 hours and deionised water (47 g, 2.60 mol) was fed at an estimated rate for 8 hours. After feeding, the reaction was left to stir at 45∼50 °C for 1 hour. A second addition of chlorine gas (25 g, 0.352 mol) was fed over 2.5 hours at 45∼50 °C and left to stir for 1 hour. The crude product was heated at 70 °C and washed with deionised water (149 g, 8.28 mol). The organic layer was separated in a glass separating funnel and added to toluene (125 g, 1.36 mol), then washed twice with a 3.5% solution of sodium chloride (149 g). A Dean Stark trap was used to strip off excess solvent and the product was set to 70.2% active ingredient in toluene resulting in 359 g (85.6%) of C₄F₉(CH₂CF₂)₂CH₂CH₂SO₂Cl.

Dimethylaminopropylamine (41 g, 0.401 mol) and toluene (62.6 g, 0.679 mol) were charged to a 3-neck round-bottom flask equipped with a reflux condenser, nitrogen inlet, addition funnel, magnetic stirrer and temperature probe. The mixture was heated to 45 °C followed by the drop wise addition of C₄F₉(CH₂CF₂)₂CH₂CH₂SO₂Cl (100 g, 0.211 mol) resulting in an exotherm. The reaction mixture was heated to 75 °C for 24 hours, filtered through a fritted glass filter with a slight vacuum and the wet cake washed with warm (60 °C) toluene (89.5 g, 0.971 mol). The solvent was evaporated under reduced pressure and the organic product was washed with warm (95 °C) deionised water (200 g, 11.1 mol), separated in a glass separating funnel and re-washed with a 4% solution of sodium chloride (200 g). Any remaining solvent was evaporated under reduced pressure to give 100 g (87.7%) of Intermediate 3, C₄F₉(CH₂CF₂)₂CH₂CH₂SO₂N(H)-CH₂CH₂CH₂N(CH₃)₂; mp 52∼58 °C.

### EXAMPLES

### Example 1

Intermediate 1, C₃F₇OCF₂CF₂CH₂CH₂SO₂N(H)-CH₂CH₂CH₂N(CH₃)₂ (7 g, 0.0146 mol), was added to a mixture of ethanol (5.4 g), deionized water (0.193 g, 0.0107 mol), sodium chloroacetate (1.74 g, 0.0149 mol) and celite (2.75 g). The reaction was refluxed overnight and filtered. The filtrate, C₃F₇OCF₂CF₂CH₂CH₂SO₂-N(H)CH₂CH₂CH₂N(CH₃)₂⁺CH2C(O)O⁻, was diluted to a 27% active ingredient with ethanol and water. The product was tested using Test Methods 1 to 3. Results are listed in Tables 2-7.

### Example 2

A mixture of Intermediate 1, C₃F₇OCF₂CF₂CH₂CH₂SO₂N(H)-CH₂CH₂CH₂N(CH₃)₂ (20 g, 0.0418 mol), and ethanol (16.7 g, 0.320 mol) was charged to a 3-neck round bottom flask equipped with a reflux condenser, nitrogen inlet, addition funnel, magnetic stirrer and temperature probe and heated to 50 °C. Hydrogen peroxide (1.75 g, 0.514 mol) was added drop wise and maintained at 50 °C for 56 hours. A second addition of hydrogen peroxide (1.75 g, 0.514 mol) was added to the reaction and maintained at 50 °C for an extra 12 hours Manganese (IV) oxide (0.004 g, 0.0000460 mol) was added gradually and held at 50 °C for an additional 16 hours. The reaction mixture was then filtered through a fritted glass filter with a slight vacuum and excess solvent was evaporated. This yielded 11.8 g (51.0%) of C₃F₇OCF₂CF₂CH₂CH₂SO₂N(H)-CH₂CH₂CH₂N⁺(CH₃)₂O⁻ that was diluted with ethanol (8.9 g, 0.193 mol) and deionised water (8.9 g, 0.494 mol) to give a 40% active ingredient concentrated solution. The product was tested using Test Methods 1 to 3. Results are listed in Tables 2-7.

### Example 3

Intermediate 2, C₄F₉CH₂CF₂CH₂CH₂SO₂N(H)-CH₂CH₂CH₂N(CH₃)₂ (7 g, 0.0147 mol), was added to a mixture of ethanol (5.4 g), deionized water (0.193 g, 0.0107 mol), sodium chloroacetate (1.74 g, 0.0149 mol) and celite (2.75 g). The reaction was refluxed overnight and filtered. The filtrate, C₄F₉CH₂CF₂CH₂CH₂SO₂N(H)-CH₂CH₂CH₂N(CH₃)₂⁺CH2C(O)O⁻, was diluted to a 27% active ingredient with ethanol and water. The product was tested using Test Methods 1 to 3. Results are listed in Tables 2-7.

### Example 4

A 3-neck round-bottom flask equipped with a reflux condenser, nitrogen inlet, addition funnel, magnetic stirrer and temperature probe was charged with Intermediate 3, C₄F₉(CH₂CF₂)₂CH₂CH₂SO₂N(H)-CH₂CH₂CH₂N(CH₃)₂ (20 g, 0.0370 mol), ethanol (13.6 g, 0.296 mol), deionised water (0.5 g, 0.0270 mol) and sodium chloroacetate (4.4 g, 0.0377 mol). The reaction mixture was heated to 78 °C for 24 hours, filtered through a fritted glass filter with a slight vacuum and the wet cake washed with warm (75 °C) ethanol (150 g, 3.26 mol). The solvent was then evaporated under reduced pressure to give 9 g (40.7%) of product C₄F₉(CH₂CF₂)₂CH₂CH₂SO₂N(H)CH₂CH₂CH₂N(CH₃)₂⁺CH₂C(O)O⁻. The final product was diluted with ethanol (11.7 g, 0.254 mol) and deionised water (12.7 g, 0.704 mol) to give a 27% active ingredient concentrated solution. The product was tested using Test Methods 1 to 3. Results are listed in Tables 2-7.

### Example 5

A mixture of Intermediate 2, C₄F₉CH₂CF₂CH₂CH₂SO₂N(H)-CH₂CH₂CH₂N(CH₃)₂ (20 g, 0.0420 mol), and ethanol (16.7 g, 0.363 mol) was charged to a 3-neck round bottom flask equipped with a reflux condenser, nitrogen inlet, addition funnel, magnetic stirrer and temperature probe and heated to 50 °C. Hydrogen peroxide (4.4 g, 0.129 mol) was added drop wise and maintained at 50 °C for 17 hours. Manganese (IV) oxide (0.0102 g, 0.000118 mol) was added gradually and held at 50 °C for an additional 16 hours. The reaction mixture was then filtered through a fritted glass filter with a slight vacuum and excess solvent was evaporated under reduced pressure. This yielded 14.3 g (69.0%) of C₄F₉CH₂CF₂CH₂CH₂SO₂N(H)-CH₂CH₂CH₂N+(CH₃)₂O⁻, which was then diluted with ethanol (10.7 g, 0.233 mol) and deionised water (10.7 g, 0.594 mol) to give a 40% active ingredient concentrated solution. The product was tested using Test Methods 1 to 3. Results are listed in Tables 2-7.

### Example 6

A mixture of Intermediate 3, C₄F₉(CH₂CF₂)₂CH₂CH₂SO₂N(H)-CH₂CH₂CH₂N(CH₃)₂ (20 g, 0.0370 mol), and ethanol (14.7 g, 0.320 mol) was charged to a 3-neck round bottom flask equipped with a reflux condenser, nitrogen inlet, addition funnel, magnetic stirrer and temperature probe and heated to 50 °C. Hydrogen peroxide (3.9 g, 0.114 mol) was added drop wise and maintained at 50 °C for 24 hours. Manganese (IV) oxide (0.009 g, 0.000104 mol) was added gradually and held at 50 °C for an additional 16 hours. The reaction mixture was then filtered through a fritted glass filter with a slight vacuum and excess solvent was evaporated. This yielded 15.2 g (74.1%) of C₄F₉(CH₂CF₂)₂CH₂CH₂SO₂N(H)-CH₂CH₂CH₂N⁺(CH₃)₂O⁻ that was diluted with ethanol (11.4 g, 0.248 mol) and deionised water (11.4 g, 0.633 mol) to give a 40% active ingredient concentrated solution. The product was tested using Test Methods 1 to 3. Results are listed in Tables 2-7.

### Comparative Example A

The procedure of the Intermediate 1 was repeated, but a perfluoroalkylethyl iodide of the formula F(CF₂)ₙCH₂CH₂I was used, wherein n ranged from 6 to 8 as the fluorinated iodide. The typical mixture was as follows: 0.68% of n=4, 67.8% of n=6, 19.5% of n=8, 7.2% of n=10, 2.4% of n=12, 0.79% of n=14, 0.23% of n=16, 0.07% of n=18 and 0.02% of n=20. The resulting product, F(CF₂)ₙCH₂CH₂SO₂N(H)-CH₂CH₂CH₂N(CH₃)₂ was tested according to Test Method 1. Results are in Table 2.

### Comparative Example B

The procedure for Intermediate 1 was repeated but used a perfluoroalkylethyl iodide of the formula F(CF₂)₆CH₂CH₂I. The resulting product intermediate was then reacted similar to Example 1. The resulting product, F(CF₂)₆CH₂CH₂SO₂N(H)-CH₂CH₂CH₂N(CH₃)₂⁺CH₂C(O)O⁻, was according to Test Methods 1 to 3. Results are in Tables 3 to 7.

### Comparative Example C

The procedure for Intermediate 1 was repeated but used a perfluoroalkylethyl iodide of the formula F(CF₂)₆CH₂CH₂I. The resulting intermediate was then reacted similar to Example 2. The resulting product F(CF₂)₆CH₂CH₂SO₂N(H)-CH₂CH₂CH₂N⁺(CH₃)₂O⁻ was tested for leveling and wetting according to the procdeure of Test Methods 1 to 3. Results are in Tables 3 to 7.

**Table 2 - Surface Tension in Deionized Water (dynes/cm) at 23 °C**

| **Example*** | **1%** | **0.5%** | **0.2%** | **0.1%** | **0.05%** | **0.01%** | **0.005%** |
|---|---|---|---|---|---|---|---|
| Example 1 | 18.2 | 15.2 | 15.6 | 17.3 | 17.9 | 20.7 | 20.3 |
| Example 2 | 15.8 | 15.7 | 17.1 | 16.1 | 16.5 | 20.0 | 21.4 |
| Example 3 | 17.7 | 18.9 | 15.9 | 18.4 | 22.6 | 25.3 | 24.4 |
| Example 4 | 18.3 | 17.9 | 16.9 | 19.2 | 16.7 | 20.6 | 27.5 |
| Example 5 | 16.1 | 18.4 | 16.9 | 17.6 | 21.2 | 19.4 | 25.1 |
| Example 6 | 16.8 | 17.8 | 16.6 | 17.9 | 17.2 | 19.4 | 19.7 |
| Comparative Example A | 17.3 | 17.3 | 17.5 | 18.0 | 18.5 | 18.1 | 20.9 |

| | | | | | | | |
|---|---|---|---|---|---|---|---|
| *Example was added to deionized water by weight based on solids of the additive in deionized water. | | | | | | | |

Normal surface tension of deionized water is 72 dyne/cm. When the above surfactants were added at a specified rate, the surface tension of each aqueous solution was reduced significantly. All the Examples 1 to 6 used in the method of the invention containing 4 or less fully fluorinated carbons showed comparable or better performance at higher concentrations to Comparative Example A containing a mixture of perfluoralkyls of 4 to 20 carbons.

**Table 3 - Leveling in RHOPLEX Floor Finish**

| **Examples** | **Reading*** |
|---|---|
| Example 1 | 3.1 |
| Example 2 | 3.1 |
| Example 3 | 3 |
| Example 4 | 3 |
| Example 5 | 3.1 |
| Example 6 | 3.2 |
| Blank | 1.2 |
| Comparative Example B | 3.1 |
| Comparative Example C | 3.2 |

| | |
|---|---|
| *Average of 5 coats | |

The fluorosurfactants of Examples 1 to 6 used in the method of the present invention containing an intervening CH₂ or O in the perfluoroalkyl chain and exhibited excellent wetting ability in a floor finish (RHOPLEX) formulation. Examples 1, 3 and 4 performed equally to Comparative Example B, and Examples 2, 5 and 6 performed equally to Comparative Example C. The comparative Examples B and C each contained a perfluoroalkyl of 6 carbons, while those of the examples 1 to 6 contained 4 perfluoroalkyls of 4 carbons or less.

**Table 4**

| Surface Tension in 2% KCl Aqueous Solution (dynes/cm) at 23 °C | | | | | |
|---|---|---|---|---|---|
| **Example*** | **0%** | **0.001%** | **0.01%** | **0.1%** | **0.5%** |
| 1 | 72.8 | 51.2 | 29.0 | 16.1 | 16.0 |
| 2 | 73.3 | 52.9 | 26.6 | 17.8 | 15.1 |
| 3 | 72.8 | 62.3 | 39.2 | 19.4 | 19.3 |
| 4 | 74.0 | 50.2 | 22.3 | 19.0 | 16.8 |
| 5 | 72.8 | 56.8 | 38.0 | 19.6 | 17.6 |
| 6 | 73.5 | 40.2 | 18.7 | 16.9 | 16.8 |
| Comparative Example B | 72.3 | 45.3 | 21.3 | 18.0 | 17.5 |
| Comparative Example C | 73.0 | 41.0 | 18.7 | 16.5 | 15.5 |

| | | | | | |
|---|---|---|---|---|---|
| *Example was added to 2% KCl aqueous solution by weight based on solids of the surfactant. | | | | | |

**Table 5**

| Surface Tension in 15% HCl Aqueous Solution (dynes/cm) at 23 °C | | | | | |
|---|---|---|---|---|---|
| **Example*** | **0%** | **0.001%** | **0.01%** | **0.1%** | **0.5%** |
| 1 | 76.0 | 53.5 | 32.5 | 17.2 | 16.9 |
| 3 | 76.1 | 59.0 | 40.0 | 19.1 | 18.5 |
| 4 | 76.5 | 50.5 | 25.6 | 19.0 | 18.8 |
| Comparative Example B | 76.0 | 52.8 | 22.1 | 19.0 | 18.8 |
| | | | | | |
| 2 | 76.0 | 50.8 | 27.2 | 16.4 | 16.3 |
| 5 | 76.1 | 61.1 | 38.1 | 18.5 | 18.0 |
| 6 | 76.3 | 51.0 | 25.0 | 16.9 | 16.3 |
| Comparative Example C | 76.6 | 42.4 | 17.0 | 16.5 | 16.3 |

| | | | | | |
|---|---|---|---|---|---|
| *Example was added to 15% HCl aqueous solution by weight based on solids of the surfactant. | | | | | |

Normal surface tension of deionized water, 2% KCl aqueous solution and 15% HCl aqueous solution is about 72-76 dyne/cm. When Examples 1 - 6 were added at a specified rate using the method of the present invention, the surface tension of each aqueous solution was reduced significantly. Better performance was obtained at higher concentrations. At the higher concentrations the fluorosurfactants of Examples 1, 3 and 4, and Examples 2, 5 and 6, used in the method of the present invention containing an intervening CH₂ or O in the perfluoroalkyl chain performed comparably to or better than Comparative Examples B and C, respectively, containing a perfluoroalkyl of 6 carbons.

**Table 6 - Foaming in Tap Water**

| **Example*** | **Foam Volume (mL)** | | | |
|---|---|---|---|---|
| | **Initial** | **t=5 min** | **t=10 min** | **t=15 min** |
| 1 | 325 | 255 | 228 | 205 |
| 3 | 255 | 170 | 155 | 140 |
| 4 | 225 | 135 | 117 | 115 |
| Comparative Example B | 225 | 130 | 120 | 120 |
| | | | | |
| 2 | 295 | 215 | 185 | 180 |
| 5 | 270 | 185 | 155 | 145 |
| 6 | 165 | 60 | 55 | 50 |
| Comparative Example C | 175 | 80 | 70 | 65 |

| | | | | |
|---|---|---|---|---|
| *Example was added to tap water by weight based on solids of the surfactant to make 100 mL 0.1 % solution | | | | |

**Table 7 - Foaming in Artificial Sea Water**

| **Example*** | **Foam Volume (mL)** | | | |
|---|---|---|---|---|
| | **Initial** | **t=5 min** | **t=10 min** | **t=15 min** |
| 1 | 300 | 225 | 195 | 175 |
| 2 | 275 | 195 | 175 | 155 |
| 4 | 200 | 125 | 100 | 55 |
| Comparative Example B | 160 | 80 | 60 | 60 |
| | | | | |
| 2 | 175 | 75 | 70 | 65 |
| 5 | 250 | 150 | 135 | 127 |
| 6 | 150 | 60 | 60 | 60 |
| Comparative Example C | 160 | 65 | 50 | 40 |

| | | | | |
|---|---|---|---|---|
| *Example was added to artificial sea water by weight based on solids of the surfactant to make 100 mL 0.1% solution. | | | | |

Foaming is an important desirable property for oilfield applications. A difference in foam height of up to 10 mL is produced by variation in the test method employed. Examples 1-6 containing an intervening CH₂ or O in the perfluoroalkyl chain showed substantially equivalent or superior performance to Comparative Examples B and C containing a perfluoralkyl of 6 carbons. The Examples demonstrated generation of equivalent or more foam initially, and sustained the foam over time.

## Claims

1. A method of providing a surface effect to a medium or substrate comprising contacting the medium or substrate with a compound of formula (I): 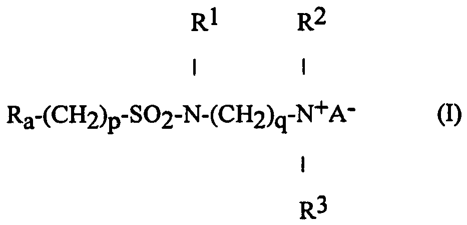 wherein
Rₐ is linear F(CF₂)ₙ(CH₂CF₂)ₘ-, or linear F(CF₂)ᵣ-O-B-;
B is (CₛF₂ₛ) optionally interrupted by 1 to 2 catenary oxygen atoms, each oxygen bonded to two carbon atoms,
n is 2 to 4, m is 1 to 4, r is 1 to 4, s is 1 to 4, provided that (r + s) is a maximum of 7,
A is O or (CH₂)ₖ-COO,
R¹ is hydrogen or a methyl,
R² and R³ are each independently alkyl having 1 to 6 carbon atoms, and p, q and k are each independently integers from 1 to 10.

2. The method of claim 1 wherein Rₐ is F(CF₂)ₙ(CH₂CF₂)ₘ-wherein n is 2 to 4 and m is 2, or wherein Rₐ is F(CF₂)ᵣ-O-B- wherein r is 3 or 4.

3. The method of claim 1 wherein the surface effect is lowering the surface tension.

4. The method of claim 3 herein the surface tension of the medium is less than 25 milli-newtons per meter at a concentration of the compound of formula (I) in the medium of less than 0.5% by weight.

5. The method of claim 3 wherein the medium is selected from the group consisting of water, saline solution, KCl solution, HCl solution, drill fluids, well fluids, liquid treatment or gas treatment stream for subterranean formation and well bore areas, hydrocarbon, halocarbon system, coating composition, latex, polymer, floor finish, floor polish, fire fighting agent, ink, emulsifying agent, foaming agent, release agent, repellency agent, flow modifier, film evaporation inhibitor, wetting agent, penetrating agent, cleaner, grinding agent, electroplating agent, corrosion inhibitor, etchant solution, soldering agent, dispersion aid, microbial agent, pulping aid, rinsing aid, polishing agent, personal care composition, drying agent, antistatic agent, and bonding agent.

6. The method of claim 1 wherein the surface effect is selected from the group consisting of wetting, penetration, spreading, leveling, flowing, emulsifying, dispersing, repelling, releasing, lubricating, etching, bonding, and stabilizing.

7. The method of claim 6 wherein the leveling and wetting are provided to a coated substrate by adding the compound of formula (I) to a coating base prior to deposition of the coating base on the substrate.

8. The method of claim 7 wherein the coating base is a water dispersed coating, alkyd coating, Type I urethane coating, unsaturated polyester coating, a floor polish, or a floor finish.

9. The method of claim 1 wherein the compound of formula (I) is added to a medium to be contacted with a hydrocarbon-bearing subterranean formation.

10. The method of claim 9 wherein the medium is selected from the group consisting of water, saline solution, KCl solution, HCl solution, hydrocarbons, halocarbons, drill fluids, well fluids, stimulating fluids, and liquid treatment or gas treatment stream for subterranean formation and well bore areas.

11. The method of claim 10 wherein the compound of formula (I) is present in the medium at a concentration of from 0.001 % to 50% by weight.

## Patentansprüche

1. Verfahren zum Bereitstellen eines Oberflächeneffekts auf einem Medium oder Substrat, umfassend das Kontaktieren des Mediums oder Substrats mit einer Verbindung der Formel (I): 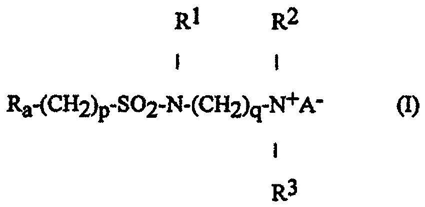 wobei
Rₐ lineares F(CF₂)ₙ(CH₂CF₂)ₘ- oder lineares F(CF₂)ᵣ-O-B- ist;
B (CₛF₂ₛ) ist, das wahlweise durch 1 bis 2 katenäre Sauerstoffatome unterbrochen ist, wobei jeder Sauerstoff an zwei Kohlenstoffatome gebunden ist,
n 2 bis 4 beträgt, m 1 bis 4 beträgt, r 1 bis 4 beträgt, s 1 bis 4 beträgt, vorausgesetzt, dass (r + s) höchstens 7 beträgt,
A O oder (CH₂)ₖ-COO ist,
R¹ Wasserstoff oder ein Methyl ist,
R² und R³ jeweils unabhängig Alkyl sind, das 1 bis 6 Kohlenstoffatome aufweist, und
p, q und k jeweils unabhängig ganze Zahlen von 1 bis 10 sind.

2. Verfahren nach Anspruch 1, wobei Rₐ lineares F(CF₂)ₙ(CH₂CF₂)ₘ- ist, wobei n 2 bis 4 beträgt und m 2 beträgt, oder wobei Rₐ F(CF₂)ᵣ-O-B- ist, wobei r 3 oder 4 beträgt.

3. Verfahren nach Anspruch 1, wobei der Oberflächeneffekt das Reduzieren der Oberflächenspannung ist.

4. Verfahren nach Anspruch 3, wobei die Oberflächenspannung des Mediums weniger als 25 Millinewton pro Meter bei einer Konzentration der Verbindung der Formel (I) in dem Medium von weniger als 0,5 Gew.-% beträgt.

5. Verfahren nach Anspruch 3, wobei das Medium aus der Gruppe ausgewählt ist bestehend aus Wasser, physiologischer Kochsalzlösung, KCl-Lösung, HCl-Lösung, Bohrfluiden, Bohrlochfluiden, Flüssigkeitsbehandlungs- oder Gasbehandlungsstrom für unterirdische Formations- und Bohrlochbereiche, Kohlenwasserstoff, Halokohlenstoffsystem, Beschichtungszusammensetzung, Latex, Polymer, Bodensiegel, Bohnerwachs, Brandlöschmittel, Tinte, Emulgiermittel, Schäumungsmittel, Trennmittel, Abweismittel, Verlaufmittel, Filmverdampfungshemmer, Benetzungsmittel, Penetriermittel, Reinigungsmittel, Schleifmittel, Galvanisiermittel, Korrosionshemmer, Ätzmittellösung, Lötmittel, Dispergierhilfsmittel, mikrobiellem Mittel, Aufschlussmittel, Spülhilfsmittel, Poliermittel, Körperpflegezusammensetzung, Trockenmittel, Antistatikmittel und Bondiermittel.

6. Verfahren nach Anspruch 1, wobei der Oberflächeneffekt aus der Gruppe ausgewählt ist bestehend aus Benetzen, Penetrieren, Ausbreiten, Egalisieren, Verlaufen, Emulgieren, Dispergieren, Abweisen, Trennen, Gleitendmachen, Ätzen, Bondieren und Stabilisieren.

7. Verfahren nach Anspruch 6, wobei das Egalisieren und Benetzen auf einem beschichteten Substrat durch Zugeben der Verbindung der Formel (I) zu einer Beschichtungsgrundierung vor dem Aufbringen der Beschichtungsgrundierung auf das Substrat bereitgestellt werden.

8. Verfahren nach Anspruch 7, wobei die Beschichtungsgrundierung eine wasserdispergierte Beschichtung, Alkydbeschichtung, Typ I-Urethanbeschichtung, ungesättigte Polyesterbeschichtung, ein Bohnerwachs oder ein Bodensiegel ist.

9. Verfahren nach Anspruch 1, wobei die Verbindung der Formel (I) einem Medium zugegeben wird, das mit einer Kohlenwasserstoff führenden unterirdischen Formation in Kontakt gebracht werden soll.

10. Verfahren nach Anspruch 9, wobei das Medium aus der Gruppe ausgewählt ist bestehend aus Wasser, physiologischer Kochsalzlösung, KCl-Lösung, HCl-Lösung, Kohlenwasserstoffen, Halokohlenstoffen, Bohrfluiden, Bohrlochfluiden, Stimulierfluiden und Flüssigkeitsbehandlungs- oder Gasbehandlungsstrom für unterirdische Formations-und Bohrlochbereiche.

11. Verfahren nach Anspruch 10, wobei die Verbindung der Formel (I) in dem Medium in einer Konzentration von 0,001 bis 50 Gew.-% vorliegt.

## Revendications

1. Procédé de fourniture d'un effet de surface à un milieu ou un substrat comprenant la mise en contact du milieu ou du substrat avec un composé de formule (I): 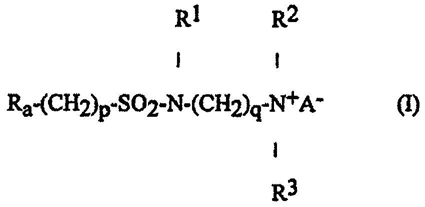 dans laquelle
Rₐ est un groupe F(CF₂)ₙ(CH₂CF₂)ₘ- linéaire, ou F(CF₂)ᵣ-O-B- linéaire;
B est (CₛF₂ₛ) optionnellement interrompu par 1 à 2 atome(s) d'oxygène caténaire(s), chaque oxygène lié à deux atomes de carbone,
n a la valeur de 2 à 4, m a la valeur de 1 à 4, r a la valeur de 1 à 4, s a la valeur de 1 à 4, à condition que (r + s) ait une valeur maximale de 7,
A est O ou (CH₂)ₖ-COO,
R¹ est un atome d'hydrogène ou un groupe méthyle,
R² et R³ sont chacun indépendamment un groupe alkyle ayant 1 à 6 atome(s) de carbone, et
p, q et k sont chacun indépendamment des nombres entiers d'une valeur de 1 à 10.

2. Procédé selon la revendication 1, dans lequel Rₐ est F(CF₂)ₙ(CH₂CF₂)ₘ-, dans laquelle n a la valeur de 2 à 4 et m a la valeur de 2, ou dans lequel Rₐ est F(CF₂)ᵣ-O-B-, dans laquelle r a la valeur de 3 ou 4.

3. Procédé selon la revendication 1, dans lequel l'effet de surface est la réduction de la tension superficielle.

4. Procédé selon la revendication 3, dans lequel la tension superficielle du milieu est inférieure à 25 millinewtons par mètre à une concentration du composé de formule (I) dans le milieu inférieure à 0,5 % en poids.

5. Procédé selon la revendication 3, dans lequel le milieu est sélectionné parmi le groupe constitué de l'eau, d'une solution saline, d'une solution de KCl, d'une solution de HCl, de boues de forage, de fluides de puits, d'écoulement de traitement des liquides ou de traitement des gaz pour les zones de formation souterraine et de forage de puits, des hydrocarbures, d'un système d'hydrocarbure halogéné, d'une composition de revêtement, du latex, d'un polymère, d'un fini pour le sol, d'un agent de polissage du sol, d'un agent d'extinction du feu, d'une encre, d'un agent émulsifiant, d'un agent de moussage, d'un agent de démoulage, d'un agent répulsif, d'un agent de modification de l'écoulement, d'un inhibiteur de l'évaporation sous forme de film, d'un agent mouillant, d'un agent de pénétration, d'un agent nettoyant, d'un agent de broyage, d'un agent d'électroplaquage, d'un inhibiteur de corrosion, d'une solution de gravure, d'un agent de soudage, d'un auxiliaire de dispersion, d'un agent microbien, d'un auxiliaire de formation de pâte, d'un auxiliaire de rinçage, d'un agent de polissage, d'une composition d'hygiène personnelle, d'un agent de séchage, d'un agent antistatique, et d'un agent de liaison.

6. Procédé selon la revendication 1, dans lequel l'effet de surface est sélectionné parmi le groupe constitué du mouillage, de la pénétration, de l'étalement, du nivellement, de la fluidification, de l'émulsification, de la dispersion, de la répulsion, du démoulage, de la lubrification, de la gravure, de la liaison, et de la stabilisation.

7. Procédé selon la revendication 6, dans lequel le nivellement et le mouillage sont fournis à un substrat revêtu par l'addition du composé de formule (I) à une base de revêtement avant le dépôt de la base de revêtement sur le substrat.

8. Procédé selon la revendication 7, dans lequel la base de revêtement est un revêtement dispersé dans l'eau, un revêtement alkyde, un revêtement uréthane de type I, un revêtement de polyester insaturé, un polissage du sol, ou un produit de finition du sol.

9. Procédé selon la revendication 1, dans lequel le composé de formule (I) est ajouté à un milieu pour être mis en contact avec une formation souterraine contenant des hydrocarbures.

10. Procédé selon la revendication 9, dans lequel le milieu est sélectionné parmi le groupe constitué de l'eau, d'une solution saline, d'une solution de KCl, d'une solution de HCl, des hydrocarbures, des hydrocarbures halogénés, des boues de forage, des fluides de puits, des fluides de stimulation, et de l'écoulement de traitement des liquides ou de traitement des gaz pour les zones de formation souterraine et de forage de puits.

11. Procédé selon la revendication 10, dans lequel le composé de formule (I) est présent dans le milieu à une concentration de 0,001 % à 50 % en poids.
